# EUROPEAN PATENT APPLICATION

(11) **EP 4 020 301 A1**
(43) Date of publication of application: **29.06.2022**
(21) Application number: 21214340.8
(22) Date of filing: 14.12.2021
(51) Int. Cl.: G06F 30/27

(54) **SIMULATING TANGIBLE MATERIAL SELECTION PROCESSES TO DETERMINE AN OPTIMAL TANGIBLE MATERIAL SELECTION PROCESS**

(30) Priority: 22.12.2020 US 202017130277
(71) Applicant: Toyota Research Institute, Inc., Los Altos, California 94022 (US)
(72) Inventor: MONTOYA, Joseph Harold, California, 94022 (US); AYKOL, Muratahan, California, 94022 (US); HUMMELSHØJ, Jens Strabo, California, 94022 (US)
(74) Representative: D Young & Co LLP

(57) **Abstract**

Material selection processes can be simulated to determine an optimal material selection process. In iterations: a process selection module (118) can select a material selection module, (120, 122, 124) a machine learning process (402) can be configured to execute the material selection module, (122, 124) the material selection module can: select information (108) about materials having known values (110) of a material property and train (B) the machine learning process (402) to produce the known values (110) in response to a receipt of the information (108) about the materials, and a measure of a performance of the material selection module (120, 122, 124) can be determined with respect to identifying a set of materials that includes the materials for which the known values are in a specific relationship with a threshold criterion for the material property. At a completion of the iterations and based on measures of performances of material selection modules, the optimal material selection process can be determined.

## Description

### TECHNICAL FIELD

The disclosed technologies are directed to simulating tangible material selection processes associated with identifying materials suitable for use in an item of manufacture in order to determine an optimal tangible material selection process.

### BACKGROUND

Material selection is a phrase used to describe processes associated with identifying materials suitable for use in an item of manufacture. Tangible material selection processes are usually performed in conjunction with other activities associated with the design of the item of manufacture. Identifying materials suitable for use in an item of manufacture can involve assessing relationships between criteria desired for the item of manufacture and material properties of candidate materials. Additionally, in situations in which the item of manufacture is intended to be made available for sale in a commercial environment, a material selection processes may also consider monetary values associated with using candidate materials in the item of manufacture. For example, such monetary values may include one or more of monetary values associated with obtaining the candidate materials or monetary values associated with incorporating the candidate materials into the item of manufacture. Because there usually are multiple criteria desired of a material for use in an item of manufacture, often several tangible material selection processes may be performed in order to identify suitable materials. For at least this reason, there can be advantages to having a systematic approach to material selection.

### SUMMARY

In an embodiment, a system for simulating tangible material selection processes to determine an optimal tangible material selection process can include one or more processors, a data store, and a memory. The data store can be communicably coupled to the one or more processors. The data store can be configured to store information about materials having known values of a material property, the known values, and a threshold criterion for the material property. The memory can be communicably coupled to the one or more processors. The memory can store a performance measurement module, a material selection process determination module, a process selection module, and a material selection module. The performance measurement module can include instructions that when executed by the one or more processors cause the one or more processors, in iterations: (1) to execute the process selection module to select the material selection module, (2) to configure a machine learning process to execute the material selection module, (3) to cause the material selection module to select information about materials having known values of a material property, (4) to cause the material selection module to train the machine learning process to produce the known values in response to a receipt of the information about the materials, and (5) to determine a measure of a performance of the material selection module with respect to identifying a set of materials that includes the materials for which the known values are in a specific relationship with a threshold criterion for the material property. The material selection module can include an electronic representation to simulate a tangible material selection process. The electronic representation can include a value for a parameter associated with the tangible material selection process. A duration of time consumed to execute the material selection module can be less than a duration of time consumed to perform the tangible material selection process. The material selection process determination module can include instructions that when executed by the one or more processors cause the one or more processors to determine, at a completion of the iterations and based on measures of performances of material selection modules, the optimal tangible material selection process.

In another embodiment, a method for simulating tangible material selection processes to determine an optimal tangible material selection process can include, in iterations: (1) executing, by a processor, a process selection module to select a material selection module, (2) configuring, by the processor, a machine learning process to execute the material selection module, (3) causing, by the processor, the material selection module to select information about materials having known values of a material property, (4) causing, by the processor, the material selection module to train the machine learning process to produce the known values in response to a receipt of the information about the materials, and (5) determining, by the processor, a measure of a performance of the material selection module with respect to identifying a set of materials that includes the materials for which the known values are in a specific relationship with a threshold criterion for the material property. The material selection module can include an electronic representation to simulate a tangible material selection process. The electronic representation can include a value for a parameter associated with the tangible material selection process. A duration of time consumed to execute the material selection module can be less than a duration of time consumed to perform the tangible material selection process. The method can include determining, by the processor at a completion of the iterations and based on measures of performances of material selection modules, the optimal tangible material selection process.

In another embodiment, a non-transitory computer-readable medium for simulating tangible material selection processes to determine an optimal tangible material selection process can include instructions that when executed by one or more processors cause the one or more processors, in iterations: (1) to execute a process selection module to select a material selection module, (2) to configure, a machine learning process to execute the material selection module, (3) to cause the material selection module to select information about materials having known values of a material property, (4) to cause the material selection module to train the machine learning process to produce the known values in response to a receipt of the information about the materials, and (5) to determine a measure of a performance of the material selection module with respect to identifying a set of materials that includes the materials for which the known values are in a specific relationship with a threshold criterion for the material property. The material selection module can include an electronic representation to simulate a tangible material selection process. The electronic representation can include a value for a parameter associated with the tangible material selection process. A duration of time consumed to execute the material selection module can be less than a duration of time consumed to perform the tangible material selection process. The non-transitory computer-readable medium can include instructions that when executed by the one or more processors cause the one or more processors to determine, at a completion of the iterations and based on measures of performances of material selection modules, the optimal tangible material selection process.

### BRIEF DESCRIPTION OF THE DRAWINGS

The accompanying drawings, which are incorporated in and constitute a part of the specification, illustrate various systems, methods, and other embodiments of the disclosure. It will be appreciated that the illustrated element boundaries (e.g., boxes, groups of boxes, or other shapes) in the figures represent one embodiment of the boundaries. In some embodiments, one element may be designed as multiple elements or multiple elements may be designed as one element. In some embodiments, an element shown as an internal component of another element may be implemented as an external component and vice versa. Furthermore, elements may not be drawn to scale.
FIG. 1 is a block diagram that illustrates an example of a system for simulating tangible material selection processes to determine an optimal tangible material selection process, according to the disclosed technologies.
FIG. 2 is a diagram that illustrates a table of example of material selection modules, according to the disclosed technologies.
FIG. 3 is a diagram that illustrates a table of examples of information about materials having known values of material properties, according to the disclosed technologies.
FIG. 4 is a diagram that illustrates an example of a first iteration of the system for simulating the tangible material selection processes to determine the optimal tangible material selection process, according to the disclosed technologies.
FIG. 5 is a diagram that illustrates an example of a second iteration of the system for simulating the tangible material selection processes to determine the optimal tangible material selection process, according to the disclosed technologies.
FIG. 6 is a diagram that illustrates a table of examples of monetary values associated with using the materials in an item of manufacture, according to the disclosed technologies.
FIG. 7 is a diagram that illustrates a table of examples of the information about the materials for which values of the material properties are to be determined, according to the disclosed technologies.
FIG. 8 is a diagram that illustrates a table of examples of the information about the materials for which the values of the material properties have been determined, according to the disclosed technologies.
FIGS. 9A and 9B include a flow diagram that illustrates an example of a method that is associated with simulating tangible material selection processes to determine an optimal tangible material selection process, according to the disclosed technologies.

### DETAILED DESCRIPTION

Because: (1) a rather large duration of time may be consumed to perform a tangible material selection process and (2) several tangible material selection processes may often be performed in order to identify materials suitable for use in an item of manufacture, there can be advantages to producing electronic representations to simulate tangible material selection processes such that durations of time consumed to execute the electronic representations are less than durations of time consumed to perform the tangible material selection processes.

The disclosed technologies can be used for simulating tangible material selection processes to determine an optimal tangible material selection process. In iterations: (1) a process selection module can be executed to select a material selection module that includes an electronic representation to simulate a tangible material selection process, (2) a machine learning process can be configured to execute the material selection module, (3) the material selection module can be caused to select information about materials having known values of a material property, (4) the material selection module can be caused to train the machine learning process to produce the known values in response to a receipt of the information about the materials, and (5) a measure of a performance of the material selection module can be determined with respect to identifying a set of materials that includes the materials for which the known values are in a specific relationship with a threshold criterion for the material property. The electronic representation can include a value for a parameter associated with the tangible material selection process. A duration of time consumed to execute the material selection module can be less than a duration of time consumed to perform the tangible material selection process. At a completion of the iterations, the optimal tangible material selection process can be determined based on measures of performances of material selection modules.

For example, the material property can include one or more of an atomic mass, an atomic number, an atomic weight, a band gap, an acoustical absorption, a speed of sound, a sound reflection, a sound transfer, a third order elasticity, an acoustoelastic effect, a corrosion resistance, a hygroscopy, a pH, a reactivity, a specific internal surface area, a surface energy, a surface tension, a capacitance, a dielectric constant, a dielectric strength, an electrical resistivity, an electrical conductivity, an electric susceptibility, an electrocaloric coefficient, an electrostriction, a magnetoelectric polarizability, a Nernst coefficient, a thermoelectric effect, a permittivity, a piezoelectric constant, a pyroelectricity, a Seebeck coefficient, a Curie temperature, a diamagnetism, a Hall coefficient, a hysteresis, a magnetostriction, a magnetothermoelectric power, a magnetic-Seebeck effect coefficient, a magnetoresistance, a permeability, a piezomagnetism, a pyromagnetic coefficient, a spin Hall effect, a castability, a machinability rating, a machining speed, a machining feed, a brittleness, a bulk modulus, a coefficient of restitution, a compressive strength, a creep, a ductility, a durability, an elasticity, fatigue limit, flexibility, flexural modulus, flexural strength, fracture toughness, friction coefficient, a hardness, a malleability, a mass diffusivity, a plasticity, a Poisson's ratio, a resilience, a shear modulus, a shear strength, a slip, a specific modulus, a specific strength, a specific weight, a stiffness, a surface roughness, a tensile strength, a toughness, a viscosity, a yield strength, a Young's modulus, an absorbance, a birefringence, a color, an electro-optic effect, a luminosity, an optical activity, a photoelasticity, a photosensitivity, a reflectivity, a refractive index, a scattering, a transmittance, a neutron cross-section, a specific activity, a half life, a binary phase diagram, a boiling point, a coefficient of thermal expansion, a critical temperature, a Curie point, a ductile brittle transition temperature, an emissivity, an eutectic point, a flammability, a flash point, a glass transition temperature, a heat of vaporization, an inversion temperature, a melting point, a specific heat thermal conductivity, a thermal diffusivity, a thermal expansion, a triple point, a vapor pressure, a specific heat capacity, or the like.

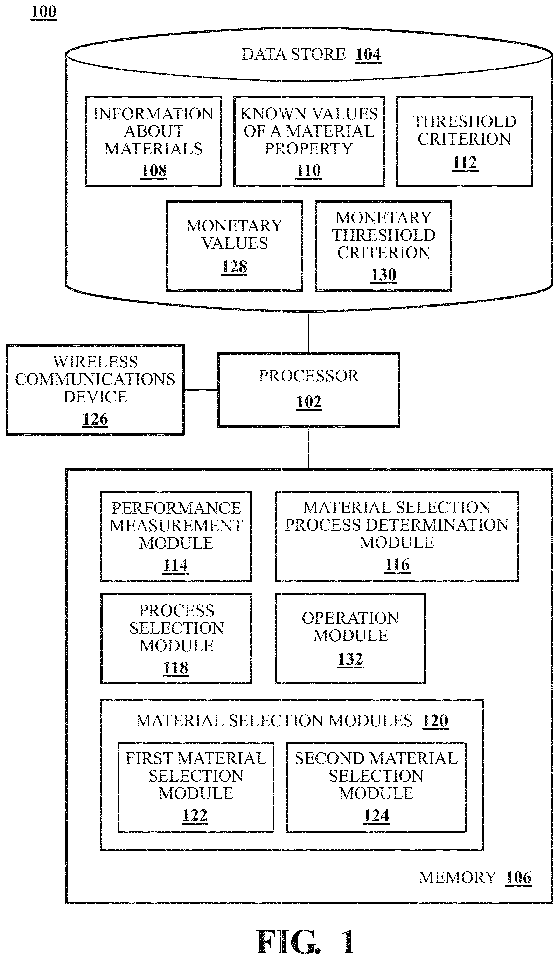

FIG. 1 is a block diagram that illustrates an example of a system 100 for simulating tangible material selection processes to determine an optimal tangible material selection process, according to the disclosed technologies. The system 100 can include, for example, a processor 102, a data store 104, and a memory 106. The data store 104 can be communicably coupled to the processor 102. For example, the data store 104 can be configured to store information 108 about materials having known values 110 of a material property, the known values 110, and a threshold criterion 112 for the material property. The memory 106 can be communicably coupled to the processor 102. For example, the memory 106 can store a performance measurement module 114, a material selection process determination module 116, a process selection module 118, and a plurality of material selection modules 120. For example, the plurality of material selection modules 120 can include a first material selection module 122 and a second material selection module 124.

Additionally, for example, the system 100 can further include a wireless communications device 126. The wireless communications device 126 can be communicably coupled to the processor 102. For example, the processor 102 can be configured to receive, via the wireless communications device 126, the threshold criterion 112. Additionally or alternatively, for example, the processor 102 can be configured to receive, via the wireless communications device 126, one or more of the information 108 about the materials or the known values 110.

The performance measurement module 114 can include instructions that function to control the processor 102, in first iterations: (1) to execute the process selection module 118 to select a material selection module (e.g., the first material selection module 122 or the second material selection module 124) that includes an electronic representation to simulate a tangible material selection process, (2) to configure a machine learning process to execute the material selection module, (3) to cause the material selection module to select information about materials having known values of a material property, (4) to cause the material selection module to train the machine learning process to produce the known values in response to a receipt of the information about the materials, and (5) to determine a measure of a performance of the material selection module with respect to identifying a set of materials that includes the materials for which the known values are in a specific relationship with the threshold criterion 112 for the material property. A duration of time consumed to execute the material selection module can be less than a duration of time consumed to perform the tangible material selection process.

The electronic representation can include a value for a first parameter associated with the tangible material selection process. For example, a process performed by the material selection module can include one or more of a simple linear regression process, a neural network process, a random forests process, a random decision forests process, a Gaussian Process Upper Confidence Bound process, a support-vector machine, an adaptive boosting process, or the like.

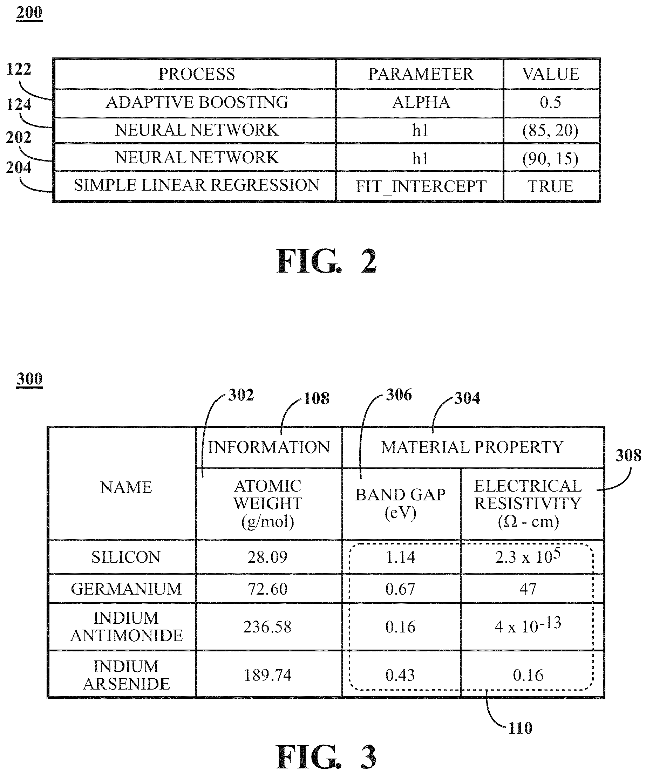

FIG. 2 is a diagram that illustrates a table 200 of example of material selection modules, according to the disclosed technologies. For example, the table 200 can include the first material selection module 122, the second material selection module 124, a third material selection module 202, and a fourth material selection module 204. For example, the first material selection module 122 can perform an adaptive boosting process and can have a value "0.5" for a parameter "alpha." For example, the second material selection module 124 can perform a neural network process and can have values "(85, 20)" for a parameter "hi." For example, the third material selection module 202 can perform a neural network process and can have values "(90, 15)" for a parameter "hi." For example, the fourth material selection module 204 can perform a simple linear regression process and can have a value "True" for a parameter "fit_intercept." Note that each of the second material selection module 124 and the third material selection module 202 can perform a neural network process with values for the parameter "h1." In this manner, for example, the first parameter, in a first iteration of the first iterations, can be identical to the first parameter in a second iteration of the first iterations, but the value for the first parameter, in the first iteration, can be different from the value for the first parameter in the second iteration.

Returning to FIG. 1, for example, the material selection module can further include a value for a second parameter. For example, the second parameter can be associated with one or more of a prediction of a value of the material property of a material, a classification of the material, a physical procedure related to a determination of the value of the material property, a heuristic process related to the determination, or the like.

FIG. 3 is a diagram that illustrates a table 300 of examples of the information 108 about the materials having the known values 110 of material properties, according to the disclosed technologies. For illustrative purposes, the table 300 can be for semiconductor materials. For example, the information 108 about the materials can include atomic weights 302 (g/mol) of the semiconductor materials. (That is, the information 108 about the materials can include a specific material property of the materials.) For example, the material properties 304 can include band gap values 306 (eV) of the semiconductor materials and electrical resistivity values 308 (Ω-cm) of the semiconductor materials.

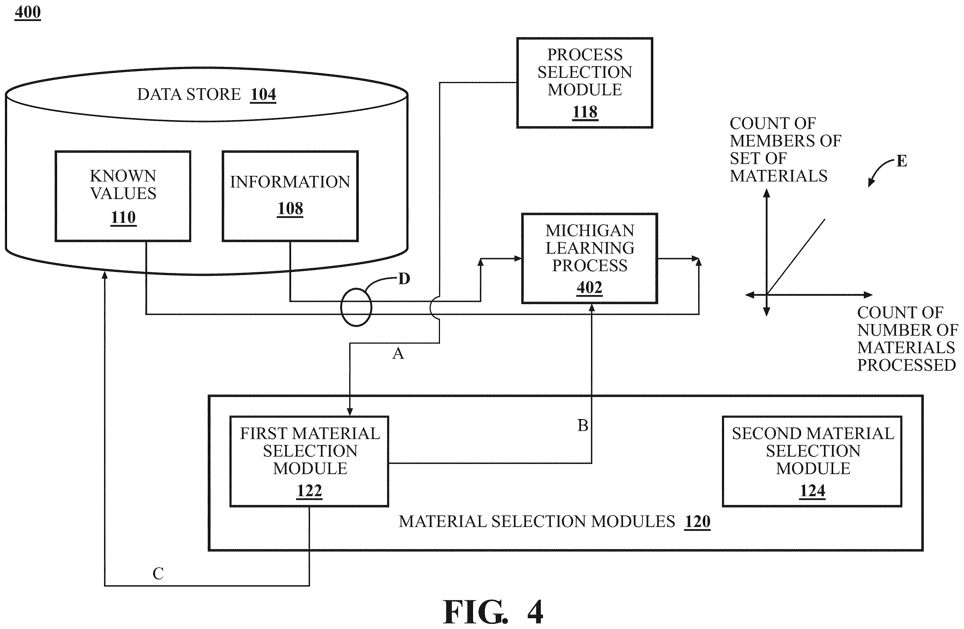

FIG. 4 is a diagram that illustrates an example of a first iteration 400, of the first iterations, of the system 100 for simulating the tangible material selection processes to determine the optimal tangible material selection process, according to the disclosed technologies. With reference to FIGS. 3 and 4, in the first iteration 400: (1) the process selection module 118 can be executed to select (A), from the plurality of material selection modules 120, the first material selection module 122 that includes an electronic representation to simulate a first tangible material selection process and performs an adaptive boosting process having the value "0.5" for the parameter "alpha," (2) a machine learning process 402 can be configured to execute (B) the first material selection module 122, (3) the first material selection module 122 can be caused to select (C), from the data store 104, the information 108 about the materials having the known values 110 of the material property 304, (4) the first material selection module 122 can be caused to train (D) the machine learning process 402 to produce the known values 110 in response to a receipt of the information 108 about the materials, and (5) a measure of a performance (E) of the first material selection module 122 can be determined with respect to identifying a set of materials that includes the materials for which the known values 110 are in a specific relationship with the threshold criterion 112 for the material property 304.

For example, the first material selection module 122 can be caused to select, from the data store 104, the atomic weights 302 (g/mol) about the materials having the known values 110 of the material property 304. For example, the first material selection module 122 can be caused to train the machine learning process 402 to produce the band gap values 306 (eV) in response to a receipt of the atomic weights 302 (g/mol). Additionally or alternatively, for example, the first material selection module 122 can be caused to train the machine learning process 402 to produce the electrical resistivity values 308 (Ω-cm) in response to a receipt of the atomic weights 302 (g/mol).

The threshold criterion 112 can be a threshold value or a threshold range of values. For example, the material property 304 can be the band gap values 306 (eV) and the specific relationship with the threshold criterion 112 can be being within a threshold range of values from 0.65 eV to 1.50 eV. For example, the material property 304 can be the electrical resistivity values 308 (Ω-cm) and the specific relationship with the threshold criterion 112 can be being greater than a threshold value of 0.1 Ω-cm.

The measure of the performance of the first material selection module 122 can be determined with respect to identifying the set of materials that includes the materials for which the known values 110 are in the specific relationship with the threshold criterion 112 for the material property 304. For example, if the material property 304 is the band gap values 306 (eV) and the specific relationship with the threshold criterion 112 is being within the threshold range of values from 0.65 eV to 1.50 eV, then the measure of the performance of the first material selection module 122 can be determined with respect to identifying that the set of materials includes Silicon and Germanium. For example, if the material property 304 is the electrical resistivity values 308 (Ω-cm) and the specific relationship with the threshold criterion 112 is being greater than the threshold value of 0.1 Ω-cm, then the measure of the performance of the first material selection module 122 can be determined with respect to identifying that the set of materials includes Silicon, Germanium, and Indium Arsenide.

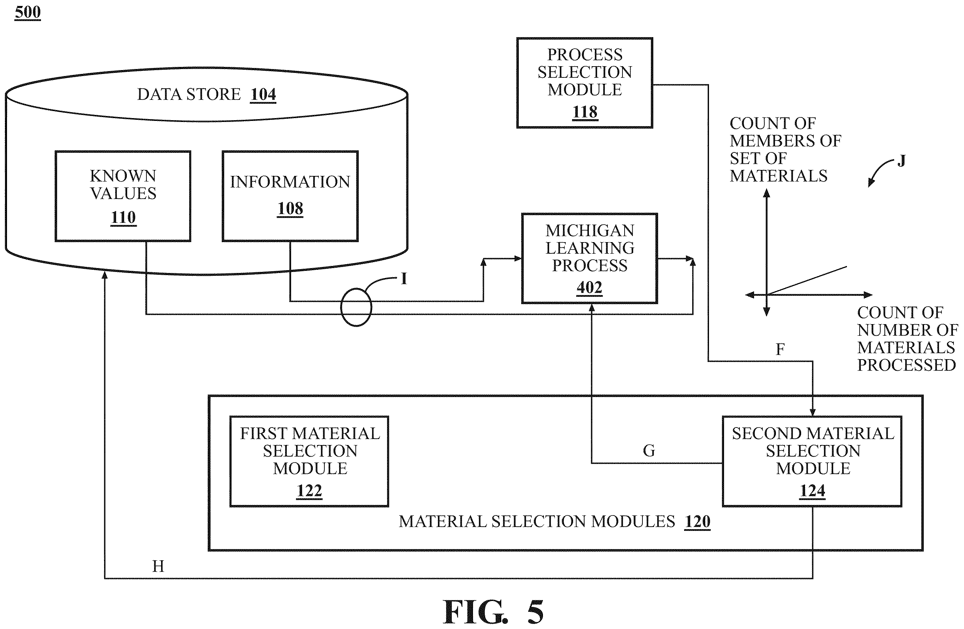

FIG. 5 is a diagram that illustrates an example of a second iteration 500, of the first iterations, of the system 100 for simulating the tangible material selection processes to determine the optimal tangible material selection process, according to the disclosed technologies. With reference to FIGS. 3 and 5, in the second iteration 500: (1) the process selection module 118 can be executed to select (F), from the plurality of material selection modules 120, the second material selection module 124 that includes an electronic representation to simulate a second tangible material selection process and performs a neural network process having the values "(85, 20)" for the parameter "hi," (2) the machine learning process 402 can be configured to execute (G) the second material selection module 124, (3) the second material selection module 124 can be caused to select (H), from the data store 104, the information 108 about the materials having the known values 110 of the material property 304, (4) the second material selection module 124 can be caused to train (I) the machine learning process 402 to produce the known values 110 in response to the receipt of the information 108 about the materials, and (5) the measure of the performance (J) of the second material selection module 124 can be determined with respect to identifying the set of materials that includes the materials for which the known values 110 are in the specific relationship with the threshold criterion 112 for the material property 304.

For example, the second material selection module 124 can be caused to select, from the data store 104, the atomic weights 302 (g/mol) about the materials having the known values 110 of the material property 304. For example, the second material selection module 124 can be caused to train the machine learning process 402 to produce the band gap values 306 (eV) in response to the receipt of the atomic weights 302 (g/mol). Additionally or alternatively, for example, the second material selection module 124 can be caused to train the machine learning process 402 to produce the electrical resistivity values 308 (Ω-cm) in response to the receipt of the atomic weights 302 (g/mol).

The threshold criterion 112 can be a threshold value or a threshold range of values. For example, the material property 304 can be the band gap values 306 (eV) and the specific relationship with the threshold criterion 112 can be being within the threshold range of values from 0.65 eV to 1.50 eV. For example, the material property 304 can be the electrical resistivity values 308 (Ω-cm) and the specific relationship with the threshold criterion 112 can be being greater than the threshold value of 0.1 Ω-cm.

The measure of the performance of the first material selection module 122 can be determined with respect to identifying the set of materials that includes the materials for which the known values 110 are in the specific relationship with the threshold criterion 112 for the material property 304. For example, if the material property 304 is the band gap values 306 (eV) and the specific relationship with the threshold criterion 112 is being within the threshold range of values from 0.65 eV to 1.50 eV, then the measure of the performance of the second material selection module 124 can be determined with respect to identifying that the set of materials includes Silicon and Germanium. For example, if the material property 304 is the electrical resistivity values 308 (Ω-cm) and the specific relationship with the threshold criterion 112 is being greater than the threshold value of 0.1 Ω-cm, then the measure of the performance of the second material selection module 124 can be determined with respect to identifying that the set of materials includes Silicon, Germanium, and Indium Arsenide.

With reference to FIG. 1, the material selection process determination module 116 can include instructions that function to control the processor 102 to determine, at a completion of the first iterations and based on measures of performances of the plurality of material selection modules 120, the optimal tangible material selection process.

With reference to FIGS. 3-5, in a first implementation, the optimal tangible material selection process can be the tangible material selection process associated with the material selection module for which the set of materials has a greatest number of members. For example, if the material property 304 is the band gap values 306 (eV) and the specific relationship with the threshold criterion 112 is being within the threshold range of values from 0.65 eV to 1.50 eV, then if the set of materials identified by the first material selection module 122 includes Silicon and Germanium, but the set of materials identified by the second material selection module 124 includes only Silicon, then the optimal tangible material selection process can be the tangible material selection process associated with the first material selection module 122. For example, if the material property 304 is the electrical resistivity values 308 (Ω-cm) and the specific relationship with the threshold criterion 112 is being greater than the threshold value of 0.1 Ω-cm, then if the set of materials identified by the second material selection module 124 includes Silicon, Germanium, and Indium Arsenide, but the set of materials identified by the first material selection module 122 includes only Silicon and Germanium, then the optimal tangible material selection process can be the tangible material selection process associated with the second material selection module 124.

In a second implementation, the instructions to cause the material selection module to select the information 108 about the materials having the known values 110 of the material property 304 and the instructions to cause the material selection module to train the machine learning process 402 to produce the known values 110 in response to the receipt of the information 108 about the materials include instructions that function to control the processor 102, in second iterations: (1) to cause the material selection module to select the information 108 about a material, of the materials, having a known value 110 of the material property 304 and (2) to cause the material selection module to train the machine learning process 402 to produce the known value 110 in response to the receipt of the information 108 about the material. The instructions to determine the measure of the performance include instructions that function to control the processor 102 to determine, at a completion of the second iterations, the measure of the performance.

For example: (1) in a first iteration of the second iterations, the first material selection module 122 can be caused: (a) to select, from the data store 104, the atomic weight 302 for Silicon and (b) to train the machine learning process 402 to produce the band gap value 306 for Silicon in response to the receipt of the atomic weight 302 for Silicon; (2) in a second iteration of the second iterations, the first material selection module 122 can be caused: (a) to select, from the data store 104, the atomic weight 302 for Germanium and (b) to train the machine learning process 402 to produce the band gap value 306 for Germanium in response to the receipt of the atomic weight 302 for Germanium; (3) in a third iteration of the second iterations, the first material selection module 122 can be caused: (a) to select, from the data store 104, the atomic weight 302 for Indium Antimonide and (b) to train the machine learning process 402 to produce the band gap value 306 for Indium Antimonide in response to the receipt of the atomic weight 302 for Indium Antimonide; and (4) in a fourth iteration of the second iterations, the first material selection module 122 can be caused: (a) to select, from the data store 104, the atomic weight 302 for Indium Arsenide and (b) to train the machine learning process 402 to produce the band gap value 306 for Indium Arsenide in response to the receipt of the atomic weight 302 for Indium Arsenide.

For example: (1) in a first iteration of the second iterations, the second material selection module 124 can be caused: (a) to select, from the data store 104, the atomic weight 302 for Indium Arsenide and (b) to train the machine learning process 402 to produce the band gap value 306 for Indium Arsenide in response to the receipt of the atomic weight 302 for Indium Arsenide; (2) in a second iteration of the second iterations, the second material selection module 124 can be caused: (a) to select, from the data store 104, the atomic weight 302 for Indium Antimonide and (b) to train the machine learning process 402 to produce the band gap value 306 for Indium Antimonide in response to the receipt of the atomic weight 302 for Indium Antimonide; (3) in a third iteration of the second iterations, the second material selection module 124 can be caused: (a) to select, from the data store 104, the atomic weight 302 for Germanium and (b) to train the machine learning process 402 to produce the band gap value 306 for Germanium in response to the receipt of the atomic weight 302 for Germanium; and (4) in a fourth iteration of the second iterations, the second material selection module 124 can be caused: (a) to select, from the data store 104, the atomic weight 302 for Silicon and (b) to train the machine learning process 402 to produce the band gap value 306 for Silicon in response to the receipt of the atomic weight 302 for Silicon.

For example, in the second implementation, the optimal tangible material selection process can be the tangible material selection process associated with the material selection module for which, at a completion of a specific number of iterations of the second iterations, the set of materials has a greatest number of members. For example, if the specific number of iterations of the second iterations is three, then if the set of materials identified by the first material selection module 122 includes Silicon and Germanium, but the set of materials identified by the second material selection module 124 includes only Germanium, then the optimal tangible material selection process can be the tangible material selection process associated with the first material selection module 122.

With reference to FIG. 1, in a third implementation, the process selection module 118 can include a process to determine the material selection module to be selected. For example, the process to determine the material selection module can include one or more of a recursion process, a historical analysis, or the like. The memory 106 can further store a process selection performance module 128. The process selection performance module 128 can include instructions that function to control the processor 102 to determine, at the completion of the first iterations and based on the measures of performances of the material selection modules 120, a measure of a performance of the process selection module 118. The process selection performance module 128 can include instructions that function to control the processor 102 to determine, at the completion of the first iterations and based on the measure of performance of the process selection module 118, an optimal material selection module. The optimal material selection module can be associated with the optimal tangible material selection process. For example, the optimal material selection module can be based on one or more of the material property or the threshold criterion 112.

With reference to FIGS. 3-5, for example, in an implementation that combines the third implementation with the first implementation, the optimal material selection module can be the material selection module for which the set of materials has the greatest number of members. For example, if the material property 304 is the band gap values 306 (eV) and the specific relationship with the threshold criterion 112 is being within the threshold range of values from 0.65 eV to 1.50 eV, then if the set of materials identified by the first material selection module 122 includes Silicon and Germanium, but the set of materials identified by the second material selection module 124 includes only Silicon, then the optimal material selection module can be the first material selection module 122. For example, if the material property 304 is the electrical resistivity values 308 (Ω-cm) and the specific relationship with the threshold criterion 112 is being greater than the threshold value of 0.1 Ω-cm, then if the set of materials identified by the second material selection module 124 includes Silicon, Germanium, and Indium Arsenide, but the set of materials identified by the first material selection module 122 includes only Silicon and Germanium, then the optimal material selection module can be the second material selection module 124.

Alternatively, in an implementation that combines the third implementation with the second implementation, the optimal material selection module can be the material selection module for which, at the completion of the specific number of iterations of the second iterations, the set of materials has the greatest number of members. For example, if the specific number of iterations of the second iterations is three, then if the set of materials identified by the first material selection module 122 includes Silicon and Germanium, but the set of materials identified by the second material selection module 124 includes only Germanium, then the optimal material selection module can be the first material selection module 122.

With reference to FIGS. 1, 4, and 5, in a fourth implementation, the data store 104 can be further configured to store one or more of monetary values 128 associated with using the materials in an item of manufacture or a monetary threshold criterion 130 for the monetary values 128. The instructions to configure the machine learning process 402 can include instructions that function to control the processor 102 to configure the machine learning process 402 to execute the material selection module to obtain the monetary values 128. The monetary values 128 can include one or more of monetary values associated with obtaining the materials, monetary values associated with incorporating the materials into the item of manufacture, or the like. For example, the processor 102 can be configured to receive, via the wireless communications device 126, one or more of the monetary values 128 or the monetary threshold criterion 130. The instructions to determine the measures of the performance can include instructions that function to control the processor 102 to determine the measure of the performance of the material selection module with respect to identifying the set of materials that includes the materials for which the monetary values 128 are in a specific relationship with a monetary threshold criterion 130. For example, the specific relationship with the monetary threshold criterion 130 can be being less than a threshold monetary value, greater than the threshold monetary value, or within a threshold range of monetary values.

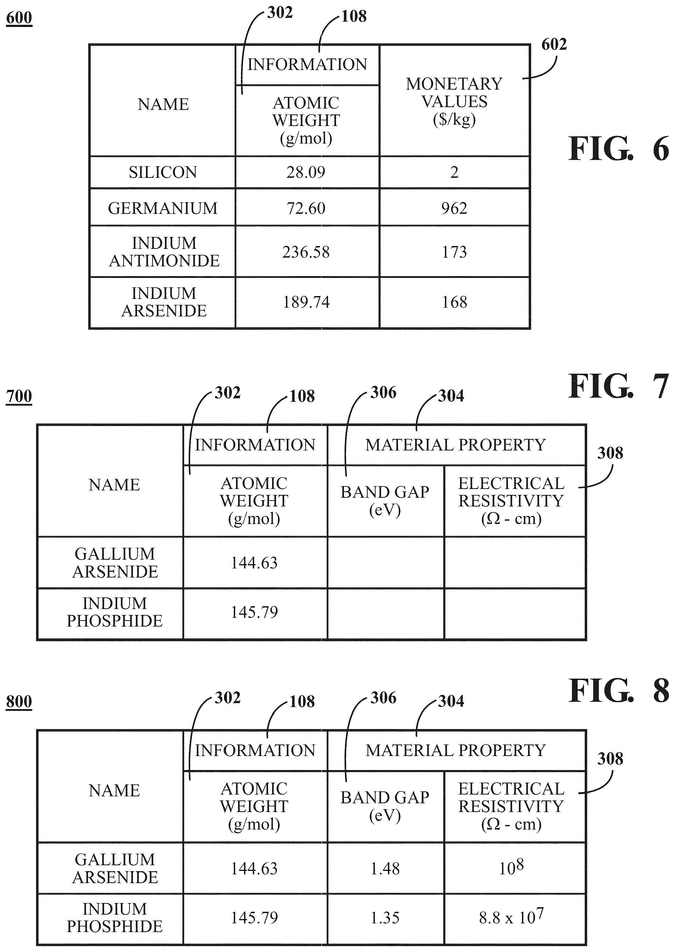

FIG. 6 is a diagram that illustrates a table 600 of examples of the monetary values 128 ($/kg) associated with using the materials in the item of manufacture, according to the disclosed technologies. With reference to FIGS. 4-6, for example: (1) the machine learning process 402 can be configured to execute the material selection module to obtain: (a) the monetary value 128 for Silicon, (b) the monetary value 128 for Germanium, (c) the monetary value 128 for Indium Antimonide, and (d) the monetary value 128 for Indium Arsenide; and (2) the measures of the performance of the material selection module can be determined with respect to identifying the set of materials that includes the materials for which the monetary values 128 are in a specific relationship with the monetary threshold criterion 130. For example, if the specific relationship with the monetary threshold criterion 130 is being less than the threshold monetary value of $200/kg, then the measures of the performance of the material selection module can be determined with respect to identifying that the set of materials includes Silicon, Indium Antimonide, and Indium Arsenide.

With reference to FIGS. 1, 3, 4, and 5, in a fifth implementation, the performance measurement module 114 can further include instructions that function to control the processor 102: (1) to configure the machine learning process 402 to execute an optimal material selection module and (2) to cause the optimal material selection module to select information 108 about a material. The optimal material selection module can be associated with the optimal tangible material selection process. The material can be different from the materials used to train the machine learning process 402. The memory 106 can further store an operation module 132. The operation module 132 can include instructions that function to control the processor 102 to cause the optimal material selection module to produce a value of the material property 304 for the material in response to a receipt of the information 108 about the material.

FIG. 7 is a diagram that illustrates a table 700 of examples of the information 108 about the materials for which values of the material properties are to be determined, according to the disclosed technologies. For illustrative purposes, the table 700 can be for semiconductor materials. For example, the information 108 about the materials can include the atomic weights 302 (g/mol) of the semiconductor materials. (That is, the information 108 about the materials can include a specific material property of the materials.) For example, the material properties 304 can include the band gap values 306 (eV) of the semiconductor materials and the electrical resistivity values 308 (Ω-cm) of the semiconductor materials.

With reference to FIGS. 1, 4, 5, and 7, for example, the machine learning process 402 can be configured to execute an optimal material selection module. For example, as described above with respect to training the machine learning process 402: (1) if the material property 304 is the band gap values 306 (eV) and the specific relationship with the threshold criterion 112 is being within the threshold range of values from 0.65 eV to 1.50 eV, then the optimal material selection module can be the first material selection module 122; and (2) if the material property 304 is the electrical resistivity values 308 (Ω-cm) and the specific relationship with the threshold criterion 112 is being greater than the threshold value of 0.1 Ω-cm, then the optimal material selection module can be the second material selection module 124. For example, the optimal material selection module can be caused: (1) (a) to select, from the data store 104, the atomic weight 302 for Gallium Arsenide and (b) to produce one or more of the band gap value 306 for Gallium Arsenide or the electrical resistivity value 308 for Gallium Arsenide and (2)(a) to select, from the data store 104, the atomic weight 302 for Indium Phosphide and (b) to produce one or more of the band gap value 306 for Indium Phosphide or the electrical resistivity value 308 for Indium Phosphide. FIG. 8 is a diagram that illustrates a table 800 of examples of the information 108 about the materials for which the values of the material properties have been determined, according to the disclosed technologies.

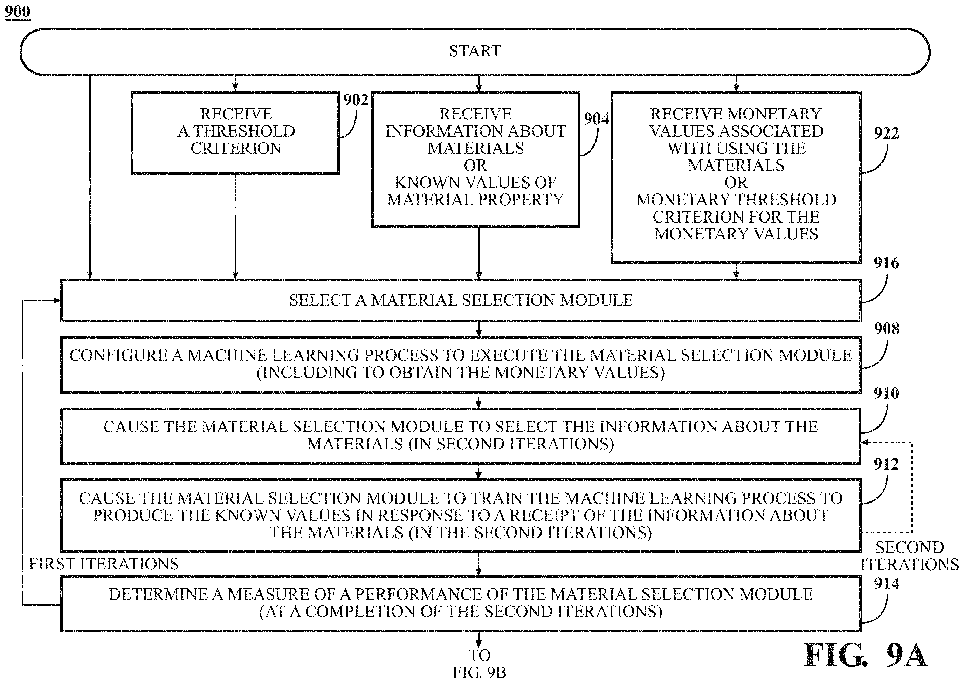

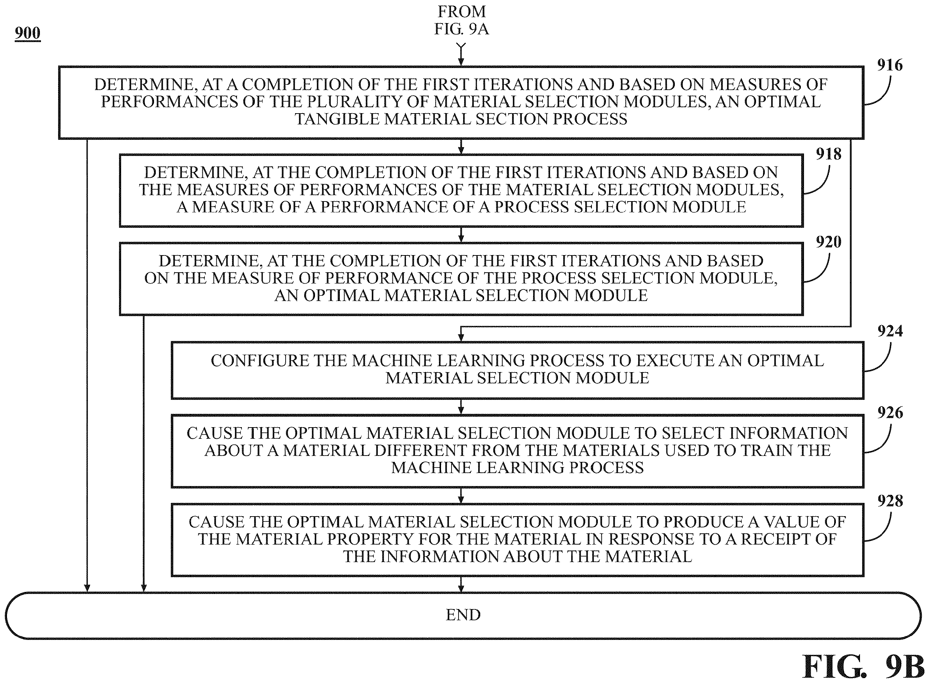

FIGS. 9A and 9B include a flow diagram that illustrates an example of a method 900 that is associated with simulating tangible material selection processes to determine an optimal tangible material selection process, according to the disclosed technologies. The method 900 is described from the perspective of the system 100 illustrated in FIG. 1. Although the method 900 is described in combination with the system 100, one of skill in the art understands, in light of the description herein, that the method 900 is not limited to being implemented by the system 100. Rather, the system 100 is an example of a system that may be used to implement the method 900.

In FIG. 9A, in the method 900, in a configuration, at an operation 902, the processor 102 can receive the threshold criterion 112 for the material property 304.

In a configuration, at an operation 904, the processor 102 can receive one or more of the information 108 about the materials or the known values 110 of the material property 304.

The performance measurement module 114 can execute, in first iterations, an operation 906, an operation 908, an operation 910, an operation 912, and an operation 914.

At the operation 906, the performance measurement module 114 can execute the process selection module 118 to select a material selection module (e.g., the first material selection module 122 or the second material selection module 124) that includes an electronic representation to simulate a tangible material selection process. A duration of time consumed to execute the material selection module can be less than a duration of time consumed to perform the tangible material selection process.

The electronic representation can include a value for a first parameter associated with the tangible material selection process. For example, a process performed by the material selection module can include one or more of a simple linear regression process, a neural network process, a random forests process, a random decision forests process, a Gaussian Process Upper Confidence Bound process, a support-vector machine, an adaptive boosting process, or the like.

For example, the first parameter, in a first iteration of the first iterations, can be identical to the first parameter in a second iteration of the first iterations, but the value for the first parameter, in the first iteration, can be different from the value for the first parameter in the second iteration. For example, the material selection module can further include a value for a second parameter associated with one or more of a prediction of a value of the material property of a material, a classification of the material, a physical procedure related to a determination of the value of the material property, a heuristic process related to the determination, or the like.

At the operation 908, the performance measurement module 114 can configure the machine learning process 402 to execute the material selection module.

At the operation 910, the performance measurement module 114 can cause the material selection module to select, from the data store 104, the information 108 about materials having the known values 110 of the material property 304.

At the operation 912, the performance measurement module 114 can cause the material selection module to train the machine learning process 405 to produce the known values 110 in response to a receipt of the information 108 about the materials.

At the operation 914, the performance measurement module 114 can determine a measure of a performance of the material selection module with respect to identifying a set of materials that includes the materials for which the known values 110 are in a specific relationship with a threshold criterion 112 for the material property 304.

In FIG. 9B, in the method 900, at an operation 916, the material selection process determination module 116 can determine, at a completion of the first iterations and based on measures of performances of the plurality of material selection modules 120, the optimal tangible material selection process.

In a first implementation, the optimal tangible material selection process can be the tangible material selection process associated with the material selection module for which the set of materials has a greatest number of members.

In a second implementation, the performance measurement module 114 can execute, in second iterations, the operation 910 and the operation 912. At the operation 910, the performance measurement module 114 can cause the material selection module to select, from the data store 104, the information 108 about a material, of the materials, having the known value 110 of the material property 304. At the operation 912, the performance measurement module 114 can cause the material selection module to train the machine learning process 402 to produce the known value 110 in response to a receipt of the information 108 about the material. At the operation 914, the performance measurement module 114 can determine, at a completion of the second iterations, the measure of the performance.

For example, in the second implementation, the optimal tangible material selection process can be the tangible material selection process associated with the material selection module for which, at a completion of a specific number of iterations of the second iterations, the set of materials has a greatest number of members.

In a third implementation, the process selection module 118 can include a process to determine the material selection module to be selected. For example, the process to determine the material selection module can include one or more of a recursion process, a historical analysis, or the like.

In the third implementation, at an operation 918, the process selection performance module 128 can determine, at the completion of the first iterations and based on the measures of performances of the material selection modules 120, a measure of a performance of the process selection module 118.

In the third implementation, at an operation 920, the process selection performance module 128 can determine, at the completion of the first iterations and based on the measure of performance of the process selection module 118, an optimal material selection module. The optimal material selection module can be associated with the optimal tangible material selection process. For example, the optimal material selection module can be based on one or more of the material property or the threshold criterion 112.

In an implementation that combines the third implementation with the first implementation, the optimal material selection module can be the material selection module for which the set of materials has the greatest number of members.

Alternatively, in an implementation that combines the third implementation with the second implementation, the optimal material selection module can be the material selection module for which, at the completion of the specific number of iterations of the second iterations, the set of materials has the greatest number of members.

In a fourth implementation, for example, at an operation 922, the processor 102 can receive one or more of the monetary values 128 associated with using the materials in an item of manufacture or the monetary threshold criterion 130 for the monetary values 128. The monetary values 128 can include one or more of monetary values associated with obtaining the materials, monetary values associated with incorporating the materials into the item of manufacture, or the like.

In the fourth implementation, at the operation 908, the performance measurement module 114 can configure the machine learning process 402 to execute the material selection module to obtain the monetary values.

In the fourth implementation, at the operation 914, the performance measurement module 114 can determine the measure of the performance of the material selection module with respect to identifying the set of materials that includes the materials for which the monetary values 128 are in a specific relationship with the monetary threshold criterion 130. For example, the specific relationship with the monetary threshold criterion 130 can be being less than a threshold monetary value, greater than the threshold monetary value, or within a threshold range of monetary values.

In a fifth implementation, at an operation 924, the performance measurement module 114 can configure the machine learning process 402 to execute an optimal material selection module. The optimal material selection module can be associated with the optimal tangible material selection process.

In the fifth implementation, at an operation 926, the performance measurement module 114 can cause the optimal material selection module to select information 108 about a material. The material can be different from the materials used to train the machine learning process 402.

In the fifth implementation, at an operation 928, the operation module 132 can cause the optimal material selection module to produce a value of the material property 304 for the material in response to a receipt of the information 108 about the material.

Detailed embodiments are disclosed herein. However, one of skill in the art understands, in light of the description herein, that the disclosed embodiments are intended only as examples. Therefore, specific structural and functional details disclosed herein are not to be interpreted as limiting, but merely as a basis for the claims and as a representative basis for teaching one of skill in the art to variously employ the aspects herein in virtually any appropriately detailed structure. Furthermore, the terms and phrases used herein are not intended to be limiting but rather to provide an understandable description of possible implementations. Various embodiments are illustrated in FIGS. 1-8, 9A, and 9B, but the embodiments are not limited to the illustrated structure or application.

The flowchart and block diagrams in the figures illustrate the architecture, functionality, and operation of possible implementations of systems, methods, and computer program products according to various embodiments. In this regard, each block in flowcharts or block diagrams may represent a module, segment, or portion of code, which comprises one or more executable instructions for implementing the specified logical function(s). One of skill in the art understands, in light of the description herein, that, in some alternative implementations, the functions described in a block may occur out of the order depicted by the figures. For example, two blocks depicted in succession may, in fact, be executed substantially concurrently, or the blocks may be executed in the reverse order, depending upon the functionality involved.

The systems, components and/or processes described above can be realized in hardware or a combination of hardware and software and can be realized in a centralized fashion in one processing system or in a distributed fashion where different elements are spread across several interconnected processing systems. Any kind of processing system or another apparatus adapted for carrying out the methods described herein is suitable. A typical combination of hardware and software can be a processing system with computer-readable program code that, when loaded and executed, controls the processing system such that it carries out the methods described herein. The systems, components, and/or processes also can be embedded in a computer-readable storage, such as a computer program product or other data programs storage device, readable by a machine, tangibly embodying a program of instructions executable by the machine to perform methods and processes described herein. These elements also can be embedded in an application product that comprises all the features enabling the implementation of the methods described herein and that, when loaded in a processing system, is able to carry out these methods.

Furthermore, arrangements described herein may take the form of a computer program product embodied in one or more computer-readable media having computer-readable program code embodied, e.g., stored, thereon. Any combination of one or more computer-readable media may be utilized. The computer-readable medium may be a computer-readable signal medium or a computer-readable storage medium. As used herein, the phrase "computer-readable storage medium" means a non-transitory storage medium. A computer-readable storage medium may be, for example, but not limited to, an electronic, magnetic, optical, electromagnetic, infrared, or semiconductor system, apparatus, or device, or any suitable combination of the foregoing. More specific examples of the computer-readable storage medium would include, in a non-exhaustive list, the following: a portable computer diskette, a hard disk drive (HDD), a solid-state drive (SSD), a read-only memory (ROM), an erasable programmable read-only memory (EPROM or flash memory), a portable compact disc read-only memory (CD-ROM), a digital versatile disc (DVD), an optical storage device, a magnetic storage device, or any suitable combination of the foregoing. As used herein, a computer-readable storage medium may be any tangible medium that can contain or store a program for use by or in connection with an instruction execution system, apparatus, or device.

Generally, modules, as used herein, include routines, programs, objects, components, data structures, and so on that perform particular tasks or implement particular data types. In further aspects, a memory generally stores such modules. The memory associated with a module may be a buffer or may be cache embedded within a processor, a random-access memory (RAM), a ROM, a flash memory, or another suitable electronic storage medium. In still further aspects, a module as used herein, may be implemented as an application-specific integrated circuit (ASIC), a hardware component of a system on a chip (SoC), a programmable logic array (PLA), or another suitable hardware component that is embedded with a defined configuration set (e.g., instructions) for performing the disclosed functions.

Program code embodied on a computer-readable medium may be transmitted using any appropriate medium, including but not limited to wireless, wireline, optical fiber, cable, radio frequency (RF), etc., or any suitable combination of the foregoing. Computer program code for carrying out operations for aspects of the disclosed technologies may be written in any combination of one or more programming languages, including an object-oriented programming language such as Java^{™}, Smalltalk, C++, or the like, and conventional procedural programming languages such as the "C" programming language or similar programming languages. The program code may execute entirely on a user's computer, partly on a user's computer, as a stand-alone software package, partly on a user's computer and partly on a remote computer, or entirely on a remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

The terms "a" and "an," as used herein, are defined as one or more than one. The term "plurality," as used herein, is defined as two or more than two. The term "another," as used herein, is defined as at least a second or more. The terms "including" and/or "having," as used herein, are defined as comprising (i.e., open language). The phrase "at least one of . . . or . . . " as used herein refers to and encompasses any and all possible combinations of one or more of the associated listed items. For example, the phrase "at least one of A, B, or C" includes A only, B only, C only, or any combination thereof (e.g., AB, AC, BC, or ABC).

Aspects herein can be embodied in other forms without departing from the spirit or essential attributes thereof. Accordingly, reference should be made to the following claims, rather than to the foregoing specification, as indicating the scope hereof.

## Claims

1. A method for simulating tangible material selection processes to determine an optimal tangible material selection process, comprising:
in iterations:
selecting, by a processor, a material selection module, having a first parameter associated with a tangible material selection process, to simulate the tangible material selection process;
configuring, by the processor, a machine learning process to execute the material selection module;
causing, by the processor, the material selection module to select information about materials having known values of a material property;
causing, by the processor, the material selection module to train the machine learning process to produce the known values in response to a receipt of the information; and
determining, by the processor, a measure of a performance of the material selection module with respect to identifying a set of materials that includes the materials for which the known values are in a specific relationship with a threshold criterion for the material property; and
determining, by the processor, the optimal tangible material selection process.

2. The method of claim 1, wherein a process performed by the material selection module includes at least one of a simple linear regression process, a neural network process, a random forests process, a random decision forests process, a Gaussian Process Upper Confidence Bound process, a support-vector machine, or an adaptive boosting process.

3. The method of claim 1, wherein the optimal tangible material selection process is the tangible material selection process associated with the material selection module for which the set of materials has a greatest number of members.

4. The method of claim 1, wherein the material property comprises at least one of an atomic mass, an atomic number, an atomic weight, a band gap, an acoustical absorption, a speed of sound, a sound reflection, a sound transfer, a third order elasticity, an acoustoelastic effect, a corrosion resistance, a hygroscopy, a pH, a reactivity, a specific internal surface area, a surface energy, a surface tension, a capacitance, a dielectric constant, a dielectric strength, an electrical resistivity, an electrical conductivity, an electric susceptibility, an electrocaloric coefficient, an electrostriction, a magnetoelectric polarizability, a Nernst coefficient, a thermoelectric effect, a permittivity, a piezoelectric constant, a pyroelectricity, a Seebeck coefficient, a Curie temperature, a diamagnetism, a Hall coefficient, a hysteresis, a magnetostriction, a magnetothermoelectric power, a magnetic-Seebeck effect coefficient, a magnetoresistance, a permeability, a piezomagnetism, a pyromagnetic coefficient, a spin Hall effect, a castability, a machinability rating, a machining speed, a machining feed, a brittleness, a bulk modulus, a coefficient of restitution, a compressive strength, a creep, a ductility, a durability, an elasticity, fatigue limit, flexibility, flexural modulus, flexural strength, fracture toughness, friction coefficient, a hardness, a malleability, a mass diffusivity, a plasticity, a Poisson's ratio, a resilience, a shear modulus, a shear strength, a slip, a specific modulus, a specific strength, a specific weight, a stiffness, a surface roughness, a tensile strength, a toughness, a viscosity, a yield strength, a Young's modulus, an absorbance, a birefringence, a color, an electro-optic effect, a luminosity, an optical activity, a photoelasticity, a photosensitivity, a reflectivity, a refractive index, a scattering, a transmittance, a neutron cross-section, a specific activity, a half life, a binary phase diagram, a boiling point, a coefficient of thermal expansion, a critical temperature, a Curie point, a ductile brittle transition temperature, an emissivity, an eutectic point, a flammability, a flash point, a glass transition temperature, a heat of vaporization, an inversion temperature, a melting point, a specific heat thermal conductivity, a thermal diffusivity, a thermal expansion, a triple point, a vapor pressure, or a specific heat capacity.

5. The method of claim 1, further comprising:
storing, by the processor, the information about the materials, the known values, and the threshold criterion, wherein:
the selecting the material selection module comprises executing a process selection module to select the material selection module,
the material selection module includes an electronic representation of the tangible material selection process,
the electronic representation includes a value for the first parameter,
a duration of time consumed to execute the material selection module is less than a duration of time consumed to perform the tangible material selection process, and
the determining the optimal tangible material selection process comprises determining, at a completion of the iterations and based on measures of performances of material selection modules, the optimal tangible material selection process.

6. The method of claim 5, wherein the first parameter, in a first iteration of the iterations, is identical to the first parameter in a second iteration of the iterations, but the value for the first parameter, in the first iteration, is different from the value for the first parameter in the second iteration.

7. The method of claim 5, wherein the material selection module further includes a value for a second parameter associated with at least one of a prediction of a value of the material property of a material, a classification of the material, a physical procedure related to a determination of the value of the material property, or a heuristic process related to the determination.

8. A system for simulating tangible material selection processes to determine an optimal tangible material selection process, comprising:
a processor; and
a memory, the memory storing:
a performance measurement module including instructions that, when executed, cause the processor to:
in first iterations:
select a material selection module, having a parameter associated with a tangible material selection process, to simulate the tangible material selection process;
configure a machine learning process to execute the material selection module;
cause the material selection module to select information about materials having known values of a material property;
cause the material selection module to train the machine learning process to produce the known values in response to a receipt of the information; and
determine a measure of a performance of the material selection module with respect to identifying a set of materials that includes the materials for which the known values are in a specific relationship with a threshold criterion for the material property; and
a material selection process determination module including instructions that, when executed, cause the processor to determine the optimal tangible material selection process.

9. The system of claim 8, further comprising a data store configured to store the information about the materials, the known values, and the threshold criterion, wherein:
the instructions to select the material selection module are included in a process selection module,
the material selection module includes an electronic representation of the tangible material selection process,
the electronic representation includes a value for the parameter,
a duration of time consumed to execute the material selection module is less than a duration of time consumed to perform the tangible material selection process, and
the instructions to determine the optimal tangible material selection process include instructions to determine, at a completion of the first iterations and based on measures of performances of material selection modules, the optimal tangible material selection process.

10. The system of claim 9, wherein:
the process selection module includes a process to determine the material selection module to be selected,
the memory further stores a process selection performance module, and
the process selection performance module includes instructions that, when executed, cause the processor to:
determine, at the completion of the first iterations and based on the measures of performances of the material selection modules, a measure of a performance of the process selection module, and
determine, at the completion of the first iterations and based on the measure of performance of the process selection module, an optimal material selection module, the optimal material selection module being associated with the optimal tangible material selection process.

11. The system of claim 10, wherein the process to determine the material selection module comprises at least one of a recursion process or a historical analysis.

12. The system of claim 10, wherein the optimal material selection module is based on at least one of the material property or the threshold criterion.

13. The system of claim 10, wherein the optimal material selection module is the material selection module for which the set of materials has a greatest number of members.

14. The system of claim 9, wherein:
the instructions to cause the material selection module to select the information about the materials having the known values of the material property and the instructions to cause the material selection module to train the machine learning process to produce the known values in response to a receipt of the information include instructions that cause the processor, in second iterations, to:
cause the material selection module to select the information about a material, of the materials, having a known value of the material property; and
cause the material selection module to train the machine learning process to produce the known value in response to a receipt of the information about the material; and
the instructions to determine the measure of the performance include instructions that cause the processor to determine, at a completion of the second iterations, the measure of the performance.

15. The system of claim 14, wherein:
the process selection module includes a process to determine the material selection module to be selected,
the memory further stores a process selection performance module, and
the process selection performance module includes instructions that, when executed, cause the processor to:
determine, at the completion of the first iterations and based on the measures of performances of the material selection modules, a measure of a performance of the process selection module, and
determine, at the completion of the first iterations and based on the measure of performance of the process selection module, an optimal material selection module, the optimal material selection module being associated with the optimal tangible material selection process, the optimal material selection module being the material selection module for which, at a completion of a specific number of iterations of the second iterations, the set of materials has a greatest number of members.

16. The system of claim 14, wherein the optimal tangible material selection process is the tangible material selection process associated with the material selection module for which, at a completion of a specific number of iterations of the second iterations, the set of materials has a greatest number of members.

17. The system of claim 8, wherein:
the instructions to configure the machine learning process include instructions that cause the processor to configure the machine learning process to execute the material selection module to obtain monetary values associated with using the materials in an item of manufacture, and
the instructions to determine the measures of the performance include instructions that cause the processor to determine the measure of the performance of the material selection module with respect to identifying the set of materials that includes the materials for which the monetary values are in a specific relationship with a monetary threshold criterion for the monetary values.

18. The system of claim 8, wherein:
the performance measurement module further includes instructions that cause the processor to:
configure the machine learning process to execute an optimal material selection module, the optimal material selection module being associated with the optimal tangible material selection process,
cause the optimal material selection module to select information about a material, the material being different from the materials used to train the machine learning process,
the memory further stores an operation module, and
the operation module includes instructions that, when executed, cause the processor to cause the optimal material selection module to produce a value of the material property for the material in response to a receipt of the information about the material.

19. A non-transitory computer-readable medium for simulating tangible material selection processes to determine an optimal tangible material selection process, the non-transitory computer-readable medium including instructions that, when executed, cause a processor to:
in iterations:
select a material selection module, having a parameter associated with a tangible material selection process, to simulate the tangible material selection process;
configure, a machine learning process to execute the material selection module;
cause the material selection module to select information about materials having known values of a material property;
cause the material selection module to train the machine learning process to produce the known values in response to a receipt of the information; and
determine a measure of a performance of the material selection module with respect to identifying a set of materials that includes the materials for which the known values are in a specific relationship with a threshold criterion for the material property; and
determine the optimal tangible material selection process.

20. The non-transitory computer-readable medium of claim 19, wherein:
the instructions to select the material selection module are included in a process selection module,
the material selection module includes an electronic representation of the tangible material selection process,
the electronic representation includes a value for the parameter,
a duration of time consumed to execute the material selection module is less than a duration of time consumed to perform the tangible material selection process, and
the instructions to determine the optimal tangible material selection process include instructions to determine, at a completion of the iterations and based on measures of performances of material selection modules, the optimal tangible material selection process.
